# EUROPEAN PATENT SPECIFICATION

(11) **EP 1 750 583 B1**
(45) Date of publication and mention of the grant of the patent: **16.07.2014**
(21) Application number: 05711966.1
(22) Date of filing: 25.01.2005
(51) Int. Cl.: A61B 6/00

(54) **SYSTEM FOR OPERATING A MEDICAL INJECTOR AND DIAGNOSTIC IMAGING DEVICE**
SYSTEM ZUR BETÄTIGUNG EINES MEDIZINISCHEN INJEKTORS UND EINER DIAGNOSTISCHEN ABBILDUNGSVORRICHTUNG
SYSTEME POUR LE FONCTIONNEMENT D'UN INJECTEUR MEDICAL ET DISPOSITIF D'IMAGERIE DE DIAGNOSTIC

(30) Priority: 11.02.2004 US 543601 P
(43) Date of publication of application: 14.02.2007
(73) Proprietor: ACIST Medical Systems, Inc., Eden Prairie, MN 55344 (US)
(72) Inventor: WILLIAMS, Robert, Fort Salonga, NY 11768 (US)
(74) Representative: Kinsler, Maureen Catherine
(86) International application number: PCT/US2005/002282
(87) International publication number: WO 2005/076810

(56) References cited:
- US-A- 4 854 324
- US-A- 4 913 154
- US-A1- 2002 123 737
- US-A1- 2002 165 445
- US-A1- 2002 183 616
- US-A1- 2003 069 498
- US-B1- 6 397 098

## Description

### BACKGROUND OF THE INVENTION

The present invention relates generally to the field of medical imaging, and more particularly to a system for operating a medical injector and diagnostic imaging device.

Imaging equipment can be used with an injection device that introduces a contrast media into the subject being examined. However, because the imaging equipment and the injection device are separate systems, each may have its own interface display device. As a result, in a control room, technicians may encounter difficulty when attempting to operate both systems through separate interface display devices. This problem is best understood with a general review of injection systems.

For example, injection systems used for the administration of contrast media for use with imaging equipment (e.g., CT, MRI, Ultrasound, Fluoroscopy, etc.) often have an injector device control interface in close proximity to an electro-mechanical injector. In some situations, the injector device control interface is adjacent to a piece of imaging equipment. Additionally, injection systems can have a remotely located device control interface. For example, the injector device control interface can be located within the corresponding imaging control room for that piece of diagnostic radiology and/or imaging equipment. Multiple user interfaces can be made necessary or advantageous based on procedural aspects or designed functions of an imaging suite. For instance, an interface can be situated patient side and in the control room free of ionizing radiation or other diagnostic energy.

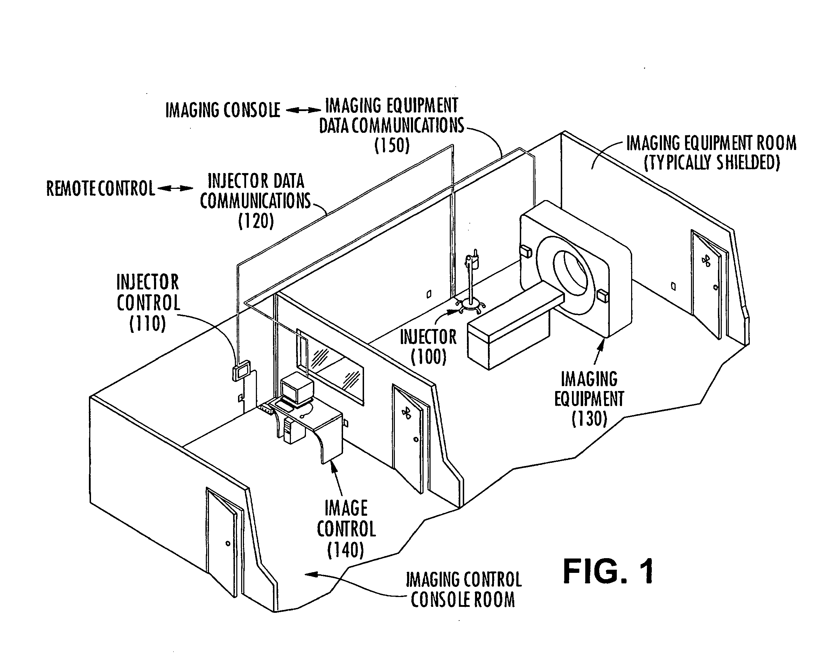

In this regard FIG. 1, illustrates a prior art injection system in use with an imaging system. Injector device **100** is coupled to injector device control interface **110** by data communications line **120,** and imaging equipment **130** is coupled to imaging device control interface **140** by data communications line **150.** Wired imaging suite remote control signals include digital, analog, TTL (Transistor-Transistor Logic) signals and or a hybrid of these signal types.

Use of user interface controls for the injector and/or imaging equipment which are in the same room as the imaging equipment is primarily, but not always, limited to features associated with patient set-up prior to, or during the early part of, exposing the patient to the energy of the imaging equipment. For the portion of the diagnostic imaging procedure in which the patient is already set-up and positioned in the imaging equipment room, clinicians program, initiate, monitor, control and terminate the imaging procedure remotely on two different interfaces (i.e., injector device control interface **110** and imaging device control interface **140**). Thus, the clinician in the imaging control room needs to concurrently, and sometimes with difficulty depending upon the clinical situation, monitor two user interfaces for the imaging and injector control units.

For various imaging procedures, there is the need to synchronize the timing of the injection to the exposure of imaging energy. For example, during a CT scan, a patient may initially be administered a specified volume of iodinated contrast media, (e.g., approximately 100 cc) at a specified flow rate (e.g., approximately 3 cc/sec) intravenously using an injector. The patient is exposed to the imaging equipment's energy at some optimum period of time after injection (e.g., within the approximate range of 10 to 45 seconds). When that optimum period occurs depends upon the fluid dynamics of the contrast media being administered to the patient by a running injector, a patient's particular physiology, and the anatomical region of interest to be imaged.

Having two user interfaces for the injector and imaging equipment places a burden upon clinicians working in the imaging suite when they attempt to achieve synchrony between the injection and imaging exposure. To address this burden, some imaging equipment manufacturers have provided connection ports on their equipment to enable connection of an injection device to an imaging device. These connection ports typically provide a TTL connection whereby limited injector and imaging equipment function is accommodated. However, the functionality of such connections is limited to synchronizing the respective start of injection to the subsequent starting of the scanner.

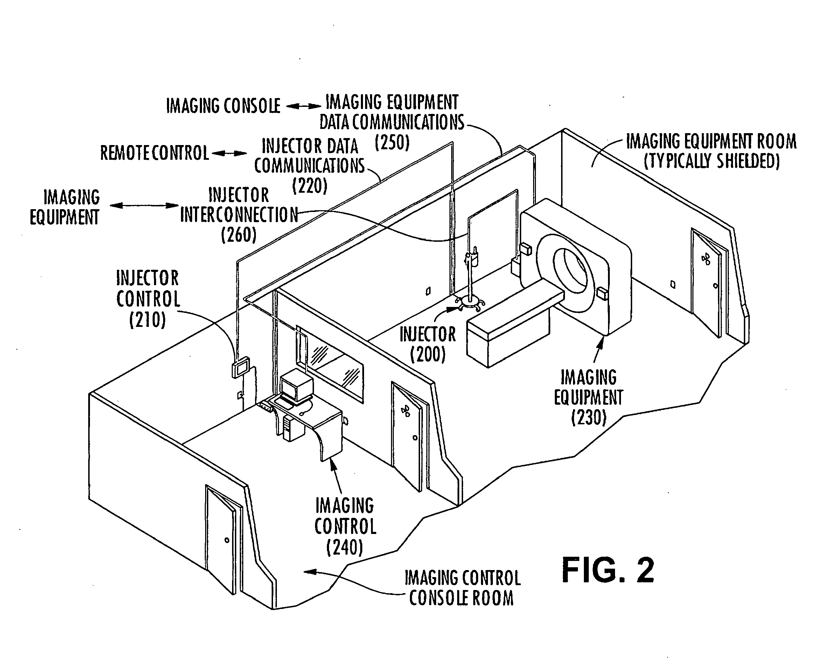

In this regard, FIG. 2 illustrates an injection device connected to an imaging device. Injector device **200** is coupled to injector device control interface **210** by data communications line **220,** and imaging equipment **230** is coupled to imaging device control interface **240** by data communications line **250.** Further, injector device **200** is also coupled to imaging equipment **230** by a signal or data communications line **260,** but data is typically only sent one-way via signal or data communications line **260** and only for synchronization of respective start times for injector device **200** and imaging equipment **230.**

Thus, there exists a need for a system whereby injection device and imaging equipment operational parameters can be controlled concurrently from a single interface or display.

US 2002/0165445 describes a system for producing a contrast-enhanced medical image of a patient. The system includes a source of contrast, a pressurizing unit, an energy source operable to apply energy to a region of the patient, an imaging unit providing a visual display of an internal view of the patient based upon a signal resulting from the energy applied to the region of the patient, and a control unit. The signal is affected by a condition of the contrast or enhancement medium in the patient. To control the procedures, the control unit adjusts the condition of the contrast or enhancement medium in the patient based upon the signal. A communication interface preferably enables information between an injector subsystem and an imaging subsystem.

US 2003/069498 describes a system for acquiring a plurality of internal images of a patient. The system includes a housing connectable to a medium movement sensor. The medium movement sensor generates a movement signal regarding a movement of the medium. The system further includes a controller secured to the housing. The controller is operable to receive the movement signal, to generate a control signal having an acquire output based on the movement signal, and to provide the control signal having an acquire output to the imaging device.

US4854324 describes an angiographic injector device for delivering contrast media to a patient at controlled rates and pressures. A processor elicits injection parameters from an operator or a pre-programmed injection module, and on the basis of the injection parameters, calculates appropriate control signals for use in a closed-loop servo system to actuate the plunger of a syringe containing the contrast media. Injection parameters includes flow rate, volume, duration, pressure limit and rise-fall time of flow rate.

US4913154 describes a medical installation for examining a patient. During exposure of the patient to an examination field, a contrast agent injector is operated to intermittently inject contrast agent into the patient at a selected frequency. An image is generated of the patient at an image pick-up frequency, and the frequency of injection of the contrast agent may be matched to, or differ from, the image pick-up frequency.

### BRIEF SUMMARY OF THE INVENTION

The present invention is defined in the accompanying independent claims. Some particular features are defined in the dependent claims. In one alternative embodiment, the present invention is directed to a system for controlling an injector device and imaging equipment from a common control console.

The common control console may include a computer or processing device that is operatively connected and in communication with an injection device and imaging device. The common control console can send and receive data to and from the injector device and the imaging equipment/device. The common control console may have a display or monitor for viewing and inputting operational commands to the injector device and the imaging equipment. The common control console may be in communication with the injection device in a wide variety of different way including, but not limited to, a wired or wireless means.

The injection device and the imaging equipment can be part of a network whereby data is shared between the control console and the injection device and the imaging equipment. Alternatively, the injection device or imaging equipment may act as an intermediary between each other and the common control console.

The injector device and the imaging equipment can individually have processing capabilities, or alternatively, can be controlled by a common processor. In one alternative embodiment of the present invention, the injector device comprises digital media comprising a software application that can be loaded onto a pre-existing imaging control console so that the injection device can be remotely controlled. In this embodiment, the software can allow the imaging control console to act as a common controller for concurrently controlling both the injector device and the imaging equipment. The software may include a wide variety of modules that can be used for controlling and optimizing the injector device.

The common control console may comprise a computer that is running under an operating system that may support a graphical user interface. Operating systems may include Windows, Linux, and the like, and any combination thereof. A graphical user interface can permit an operator to manage and run multiple programs concurrently. For example, in one embodiment of the present invention, the common control console may have an interface for the injection device and an interface for the imaging equipment that are displayed concurrently. As a result, the operator can operate and control an injection device and imaging equipment concurrently. Additionally, the common control console can store and retrieve protocols that can be used for operating the devices and imaging equipment. Such protocols may include operating parameters that can be grouped together for conducting specific tests, such as a CT scan, for example. Combined protocols can be created containing operational instructions for both the injection device and the imaging equipment. The protocols can help improve the efficiency and quality of the testing. Operational parameters for an injector include, without limitation, flow rate, media, volume, pressure, phase, keep vein open (KVO), pause, hold, delay, start, and stop. Operational parameters for an imaging device include, without limitation, tube current, tube voltage, collimation, pitch, detector configuration, rotation, pause, scan delay, start, and stop.

The present invention comprises a system for concurrently controlling both an injector device and imaging equipment. The invention also provides a system for monitoring and controlling the equipment on a common display. The invention additionally provides a system for creating stored protocols that can be used to operate both the injection device and the imaging equipment. Other features of the present invention are set forth in the drawings and detailed description.

### BRIEF DESCRIPTION OF THE SEVERAL VIEWS OF THE

### DRAWING(S)

Having thus described the invention in general terms, reference will now be made to the accompanying drawings, which are not necessarily drawn to scale, and wherein:
Figure 1 is a pictorial illustration of a prior art injection system in use with an imaging system within an imaging suite;
Figure 2 is a pictorial illustration of a prior art injection device connected to an imaging device within an imaging suite;
Figure 3 is an example and not part of the
   invention showing a pictorial illustration of an imaging device and an injector device that share an imaging/injector control console within an imaging suite,
Figure 4 is a non-limiting, block diagram of a system in which an injector and an imaging device are controlled by a common controller that is in accordance with the present invention;
Figure 5 is a non-limiting, block diagram of two system designs wherein either the injector or the imaging equipment acts as an intermediary and which is not part of the present invention;
Figure 6 is a non-limiting, block diagram of a system in which the injector and imaging equipment controller, the injector, and the imaging equipment communicate utilizing a network in accordance with at least one alternative embodiment of the present invention;
Figure 7 is a non-limiting, block diagram of a system in which multiple imaging suites each containing at least one injector and imaging equipment are networked together in accordance with at least one alternative embodiment of the present invention;
Figure 8 is a non-limiting, block diagram of a control system architecture in accordance with at least one alternative embodiment of the present invention;
Figure 9 is a non-limiting, pictorial illustration of a marketed injector system for an imaging suite that includes an injector system and software and not part of the present invention;
Figure 10 is a non-limiting, block diagram of a system with a single computing device used for imaging suite control of both the injector and imaging equipment wherein control software is provided on a storage media in accordance with at least one alternative embodiment of the present invention;
Figure 11 is a non-limiting, block diagram of an injection system utilizing a network appliance in accordance with at least one alternative embodiment of the present invention;
Figure 12 is a non-limiting, pictorial illustration of an example of how a network appliance can be deployed;
Figure 12A is a non-limiting, pictorial diagram of an example of how a injector and imaging equipment can both be considered network appliances;
Figure 13 is a non-limiting, pictorial diagram of an injector/imaging equipment console concurrently displaying one dedicated display region for the injector and another dedicated display region for the imaging equipment in accordance with at least one alternative embodiment of the present invention;
Figure 14 is a non-limiting, block diagram of a system in which both the injector control application and imaging equipment control application concurrently run on a single computer platform with sufficient processing resources, operating system capability connectivity ports and an optional dedicated control unit possessing specified control functions that service only the imaging equipment, service only the injector or both the injector and imaging equipment and which is not part of the present invention;
Figure 15 is a non-limiting, block diagram of an interface arrangement and which is not part of the present invention;
Figure 16 is a non-limiting, block diagram of a software architectural arrangement wherein the user interface application program is inclusive of both injector and imaging equipment attributes in accordance with at least one alternative embodiment of the present invention;
Figure 17 is a non-limiting, pictorial illustration of a display area of a common injector/imaging equipment console in which a single display window containing both user interface functions of the injector and associated imaging equipment in accordance with at least one alternative embodiment of the present invention;
Figure 18 is a non-limiting, pictorial illustration of an injector system utilizing a web browser and which is not part of the present invention;
Figure 19 is a non-limiting, diagram of consolidated stored procedures on a single user interface wherein separate display processes for the injector and imaging equipment exist in accordance with at least one alternative embodiment of the present invention; and
Figure 20 is a non-limiting, diagram of a consolidated store procedures on a single user interface with one display process servicing both injector and imaging equipment and stored procedure on that interface containing both injector and imaging equipment operational parameters in accordance with at least one alternative embodiment of the present invention.

### DETAILED DESCRIPTION OF THE INVENTION

The present invention now will be described hereinafter with reference to the accompanying drawings. The invention may be embodied in many different forms and the drawings and descriptions herein should not be construed as limited to the embodiments set forth herein. Like numbers refer to like elements throughout. As used herein, the term "exemplary" refers to a non-limiting alternative embodiment of the invention.

In one alternative embodiment, the invention is directed to a system for operating a medical injector and a diagnostic imaging device from a single interface or display. The injection/imaging system may comprise an injector system and imaging system that are in communication with and operatively controlled by a common imaging control console or common interface device.

An injector system may include an injector device that can be used to administer an effective dosage of a contrast medium and a control interface that is operatively connected to the injector device. The injector system may have one or more control interfaces. The control interface may send and receive data to and from the injector device. The injector device can be any type of injector mechanism that is used to deliver a contrast medium into a patient or subject (e.g., E-Z-EM EMPOWER CT Injector). The imaging system may be comprised of an imaging control console, an imaging device or equipment that can be used to monitor and display the contrast medium within a patient or subject, acquire internal images of a patient or subject, and to provide other diagnostic data to a control console or storage media. The imaging system may have an imaging interface that may be operatively connected to the imaging equipment.

The term "contrast medium" includes any suitable medium, that can be injected into an individual or subject to highlight and/or identify selected areas of the individual's body. Contrast mediums may include, but are not limited to saline media, flush media, and the like, and any combination thereof. A contrast medium may be used in conjunction with an imaging device that is used to perform medical diagnostic imaging such as CT scans, MRI, ultrasound, etc.

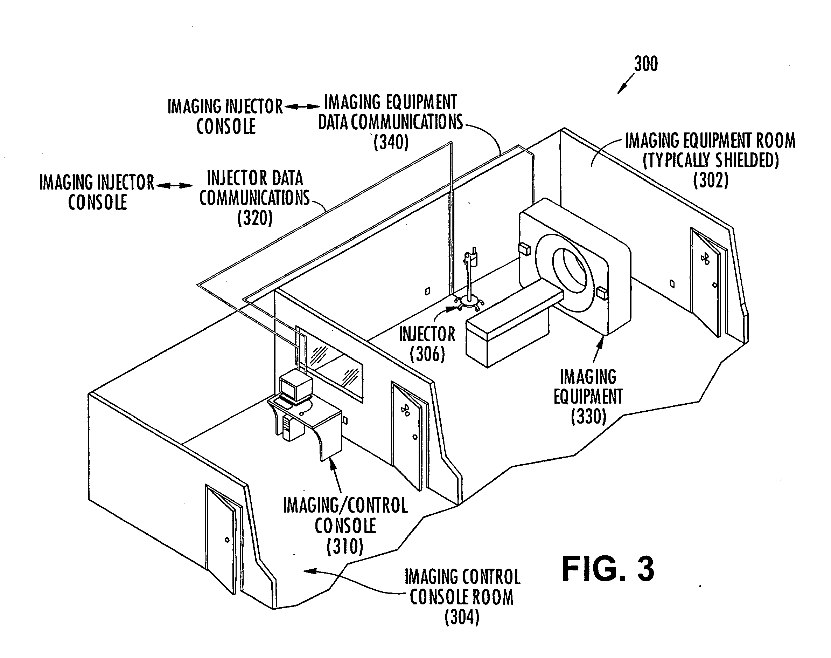

With reference to **FIG. 3****,** an example of a medical imaging suite is shown. As shown in **FIG. 3****,** the imaging suite **300** may include common control console room **304** and an imaging equipment room **302.** The imaging equipment room may comprise an imaging equipment device **330** and an injector device **306.** The imaging equipment device **330** and the injector device **306** can be in communication with, and operatively controlled by, a common control console **310.** The common control console can be in communication with devices **306, 330** in a wide variety of manners. As shown in **FIG. 3****,** the devices **306, 330** are each respectively in communication with the control console via communication channels **320, 340.** In embodiments where the imaging equipment produces a magnetic field, the communication channels between the devices and the control console and any additional devices may be adapted to be substantially non-reactive with the magnetic field of the invention. Such substantially non-reactive communication channels include, for example, fiber optic lines, an electromagnetic transmitter/receiver such as an infrared, and the like, and any combination thereof. Additionally, in the embodiments where the imaging equipment produces a magnetic field, the devices such as the injector in the imaging equipment room may comprise a material, such as brass, that is substantially non-reactive with the magnetic field. In other embodiments, the devices in the imaging equipment room may be oriented within the room so that they do not substantially interfere with the imaging equipment.

The common control console can be used to remotely control both the injector device and the imaging device from the imaging control console room. The common control console can be an imaging control console that has been modified so that it can also remotely operate an injector device. The modified control console can concurrently control both the injection device and the imaging equipment. The imaging control device may be modified by the addition of software and/or hardware. The common control console can send and receive data to and from the injector device. The terms "remote," "remotely controlled," and located "remotely" as defined herein, include components that are not in physical contact with one another, not operably engaged with one another, and/or not co-located in the same room but that may nonetheless be in communication electronically, mechanically, and/or electromechanically via a number of different communication techniques including, but not limited to, wireless connectivity means such as Bluetooth^{®}, a computer network that may link the various control components with the injector device, imaging device, or other medical devices that may be located either within or outside the medical imaging suite.

The injector device and the imaging device can also share a single processing system, or alternatively, both the injector and imaging device can have a separate processing system. In one alternative embodiment of the present invention, if both devices have a processing system, a single system may be used to control both devices. For instance, one system may have a software platform that allows it to be remotely controlled by the other computing system. In this embodiment, for example, an operator can remotely establish and monitor the injection and imaging procedure from a single user interface. In one alternative embodiment, this system could either be proprietary or open systems computing architecture that uses a commercially available computing platform (e.g., a PC architecture running Windows or a similar operating system). Within the context of the invention, an open systems computing architecture refers may include a non-specific hardware and operating software combination with no pre-specified function as it relates to control of any injector or imaging equipment or any other device, medical or otherwise. Open systems may encompass a processing unit and input-output devices such as, for example, a display, keyboard, and pointing devices such as a mouse. An operating system may include current open system computing architecture. In another alternative embodiment, the operating system software may provide a generic easily interpretable interface limited to performing basic functions of the computing platform itself and low level software routines establishing function of internal circuitry not specific to any application such as the one presented in various embodiments of this invention. The present invention may be directed to a dedicated application for operation of an injector and imaging equipment system on a common display.

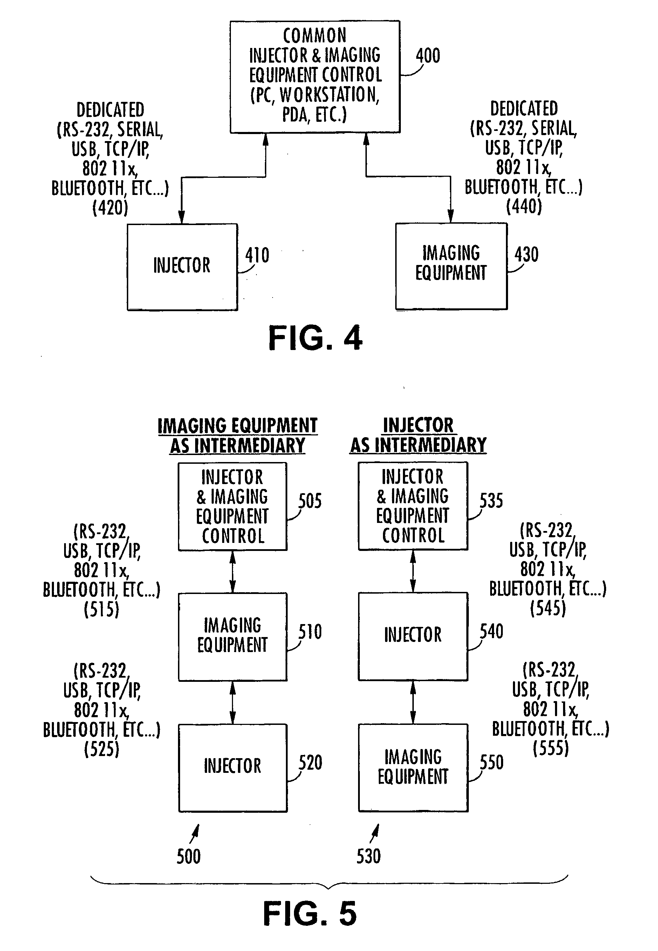

In one embodiment, a single computing system may be used to run multiple processes, including a first process for the imaging equipment, and a second for the injector device. The present system can be used to control both the imaging equipment and the injector device concurrently through a single interface. In this regard, **FIG. 4** is a block diagram illustrating that the both the injector device **410** and the imaging equipment **430** can be controlled through a common interface **400.**

The common control console may include an operator interface for providing operator control over device functions of the imaging equipment and the injector. The operator interface may include a display unit for displaying injector device and imaging equipment data such as operational controls, device status, acquired images, and the like, and any combination thereof. The display unit typically may include any type of device that can be used to output and display data, images, programs, and the like, and any combination thereof, in a format that can be read by an operator. Such devices may include, without limitation, computer and television monitors, LCD displays, plasma displays, video displays, and the like. The display means can also include an input device such as a touchscreen. The display can be used to view images and control functions that can be used to concurrently operate multiple devices.

In another alternative embodiment, the common control console may comprise a commercially available computing system such as a pc. Other computing systems and devices such as a PDA (personal digital assistant) could be used to control both the injector and imaging devices. The common control console may include multiple inputs and outputs for sending and receiving data to and from the injector device and imaging equipment. Such inputs may include, without limitation, keyboards, touch screens, buttons, pointer controls such as a mouse, voice recognition software, a dedicated controller, and the like, and combinations thereof. The common control console may also include a storage medium (e.g., magnetic, optical, printed media, or otherwise) for storing images, statistics, device operational parameters, data, error logs, personal notes, and the like, and combinations thereof.

In another alternative embodiment, the control console and the injector and imaging devices can be operatively connected and in communication with each using both wired and wireless communication protocols. Such communication protocols include but are not limited to serial communication protocols such as I2C, ACCESS.bus, RS-232, universal serial bus (USB), IEE-488(GPIB), LAN/Internet protocols such as TCP/IP, wireless protocols such as 802.11 x, and Bluetooth, etc. The communication protocols can also include proprietary systems. The control console can also be connected to the devices with a dedicated communication channel. In this regard, **FIG. 4** illustrates that the system may include dedicated communication channels **420, 440** that can be used to connect the common control console to the devices. Alternatively, the injector and the imaging equipment can be in communication with the common control console using different communication protocols. For instance, a serial data communication channel can be used to transfer data between the common control console and the injector device, and a TCP/IP network could be used for transferring data between the common control console and the imaging equipment.

In an example and not part of the present invention, the injector device or imaging equipment can also act as an intermediary, enabling a common control console to communicate with the injector through the imaging equipment or visa versa. In this regard, **FIG. 5** illustrates two alternative system designs wherein either the injector or imaging equipment can act as an intermediary. In system **500,** the common control console **505** is in direct communication with the imaging equipment **510** via communication channel **515.** The imaging equipment **510** is in turn in direct communication with the injector device **520** via communication channel **520.** In system **530** the control console **535** is in direct communication with the injector device **540,** which is in turn in direct communication with the imaging equipment **550.** The injector device and the imaging equipment can also each separately possess process capabilities. As such, each device can handle data on behalf of the other device as a communications hub or intermediary. In other alternative embodiments of the present invention, both the injector and the imaging equipment may have architectures and processing capabilities that can be programmed to process application specific data before, during and after a transmission to the control console.

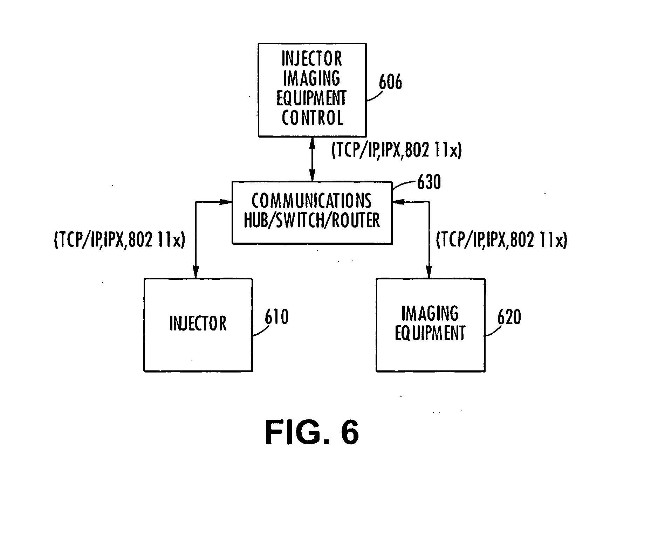

In an alternative embodiment of the present invention, the imaging equipment, injector device, and the common control console can be operatively connected and in communication with each other through a network environment. In such an environment, an independent networking device, such as a hub, switch, or router, is typically used to interconnect the control console and the devices. In this regard, **FIG. 6** illustrates a system in which a networking device is used to facilitate communication between the individual devices and control console. As shown in **FIG. 6****,** common control console **606** is in communication with the injector device **610** and the imaging equipment **620** via a networking device **630.** In the illustrated embodiment, data from the injector device and imaging equipment can be concurrently displayed on a single operator interface, and the data is communicated to the hub using a common communication protocol (e.g., wired or wireless).

In another alternative embodiment of the present invention, the networking system that is used to interconnect the devices and the control console can be chosen from a wide variety of network formats. Networking formats may include, without limitation, LAN (local-area network), WAN (wide-area network), CAN (campus-area network), WWW (world wide web), and the like, and combinations thereof. The network topology of the devices can also be varied depending upon a designer's preference. Network topographies may include, but are not limited to, bus topology, ring topology, star topology, and the like, and combinations thereof.

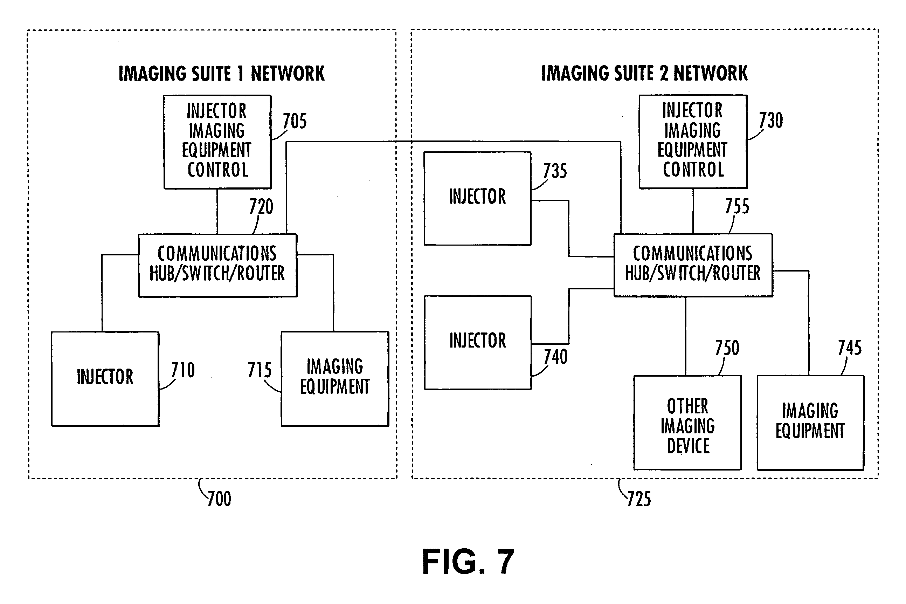

With reference to **FIG. 7****,** a system that is comprised of multiple imaging suites is illustrated. In imaging suite **700,** a common control console **705** is shown interconnected to an injector device **710** and imaging equipment **715** using a communications networking device **720.** Alternatively, multiple imaging suites can be interconnected through a network or networking device. In this regard, **FIG 7** illustrates that imaging suite **700** can be operatively connected to a second imaging suite **725.** As shown in **FIG. 7****,** networking device **720** is in communication with a second networking device **755** that is located in a separate imaging suite **725.** Multiple imaging suites can be networked together and controlled via any number of common control consoles. In one example, the control console and the imaging equipment and injector devices can all be connected on a common subnet, which is a portion of a network that shares a common address component. For example, on TCP/IP networks, such as the Internet, subnets are defined as all devices whose IP addresses have the same prefix. Thus, an operator connected on the same subnet as the control console and the network of imaging/injector devices could control and access the devices.

**FIG. 7** also illustrates that a common control console can be used to control multiple imaging devices and/or injector devices. In this regard, **FIG. 7** shows an imaging suite **725** having a common control console **730** that is operatively connected to multiple injector devices **735, 740** and multiple imaging equipment devices **745, 750.** As shown in **FIG. 7****,** the multiple devices are networked to a common control console **730** using a networking device such as a hub, router, or switch. The control console **730** may comprise a single interface that allows an operator to control an injection device and imaging equipment device concurrently. It should also be recognized that the control console 730 could be used to control multiple injector devices and imaging equipment devices in the absence of a network. In such a system, the devices could be in direct communication with the common control console, or could be routed indirectly through one of the devices, which would be acting as an intermediary.

The present invention may also provide various computer program product embodiments capable of executing various protocols for operating the injector device and the imaging equipment. In one alternative embodiment, the computer program products are capable of controlling the injector device from a remote location. The computer program product may comprise an executable portion for receiving user input from an input device.

In one embodiment, the injector device may be bundled as a package that includes the injector device and remote computer program product or hardware that can be used in conjunction with an existing imaging control console. The remote computer program product allows the imaging control console to be operatively connected to both the imaging equipment and the injector device. As a result, in one alternative embodiment of the invention, the injector device may be distributed with the computer program without the need for an associated injector control console. The common control console may include a control system architecture that can be used to control, display, analyze, and monitor the various imaging and injection devices. The control system architecture may also include hardware and software elements. With respect to the computer program product described herein, it should be recognized that there exists a wide variety of platforms and languages for creating software for performing the procedures outlined herein. It should also be recognized that the choice of the exact platform and language is often dictated by the specific requirements of the actual system being constructed. The computer program product typically includes modules or elements that are used to remotely control the injection device.

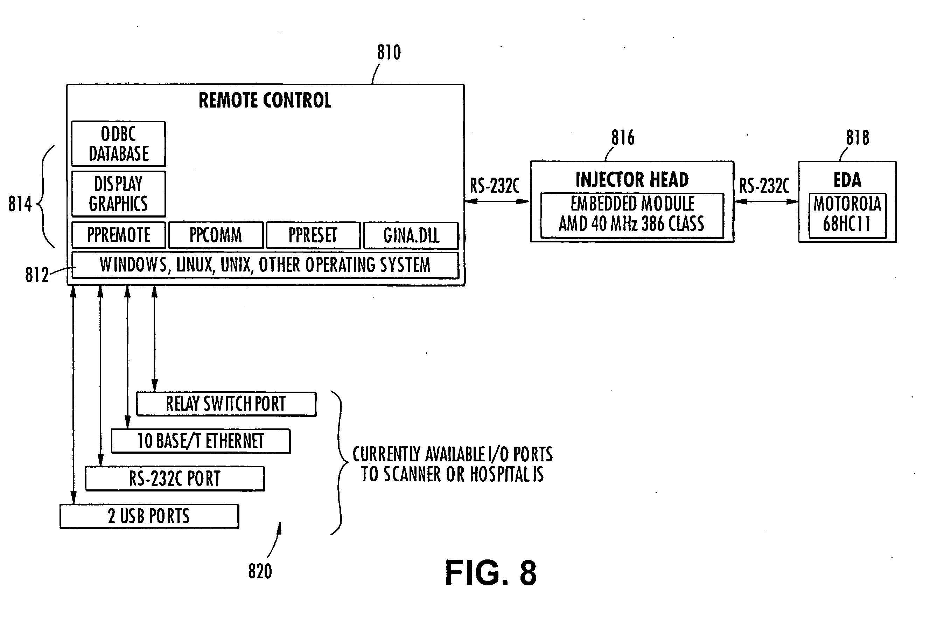

With reference to **FIG. 8****,** an exemplary control system architecture, as found on a E-Z-EM EmpowerCT™ CT Injector is illustrated. As shown, the control system architecture may include multiple executable program modules, collectively referred to as reference number **814.** The executable program modules **814** may be present on the common control console or on a hardware device **810** that is operatively connected to the common control console. In this regard, **FIG. 8** illustrates a remote control **810** having executable program modules that is operatively connected to both the injector **816** and a scanning device. The remote control may also include multiple I/O connections **820** for communicating with various networks and devices including the scanner, imaging display device, hospital network, and the like, and combinations thereof. In some embodiments of the present invention, the common control console may be further adapted to be capable of communicating with an extravasation detection device (EDA) **818** that may be located within the procedure room (such as an imaging room **302**) (see generally, **FIG. 3**) so to be capable of being operably engaged with a patient receiving an injection of media from the injector device **816.** The EDA **818** may also be in communication with the injector device **816,** remote control **810,** imaging display, and/or other computer devices via a wired and/or wireless computer network. Furthermore, the remote control **810** may also be configured to be capable of transmitting and/or receiving an extravasation data set from the EDA **818.** Although **FIG. 8** illustrates the remote control being operably connected to the injector device and EDA via a RS-232C serial communication protocol, it should be recognized that the devices, remote control, and imaging control console may be connected using a multitude of different protocols including serial communication protocols such as I2C, ACCESS.bus, RS-232, universal serial bus (USB), IEE-488(GPIB), LAN/Internet protocols such as TCP/IP, wireless protocols such as 802.11x, and Bluetooth, and the like, and any combination thereof.

As shown in **FIG. 8****,** the control system architecture can be comprised of a wide variety of executable program modules **814** that allow the common control console to remotely control devices, such as the injector. The executable program modules may include routines, programs, components, data structures and the like and any combination thereof that perform particular tasks or implement particular data types. Modules may include, without limitation, PPREMOTE, display graphics, ODBC Database, PPCOMM, PPRESET, and GINA.DLL, and the like, and any combination thereof. These modules are discussed below. The modules may operate within an operating system layer such as Windows, Unix, Linux, MACOS, and the like, and any combination thereof.

PPREMOTE includes to an executable program module or base software application that is capable of execution and running on a process on the control console. The PPREMOTE comprises the user interface visual elements on a display and accepts user input (e.g., keyboard, mouse, touchscreen, etc.). This executable program may also include program routines for storing, managing and mathematically operating on data variables that are relevant to the operation of the injector both in volatile and non-volatile memory. Inclusive of such data management function are routines to read and write to ODBC database files. This module may also transfer as well as share data to and from the PPCOMM module as required during various junctures of injector operation.

Display Graphics may comprise a library of visual elements that are selectively accessed and used by the PPREMOTE to produce user interface displays. Visual elements may include, but are not limited to, text, touch panel buttons, help files, help graphics, icons, animation, and the like, and any combination thereof. The visual elements may comprise individual image files.

OBDC Database files may be created and operated upon by a PPREMOTE process. OBDC database files can store archival data, for example, on injector diagnostics, error conditions, usage statistics, EDA performance, EDA bio-impedance profiles, user saved injection protocols, foreign language messages, etc., or any combination thereof. Such files may be stored on read-writable media such as, for example, magnetic storage devices, including hard disk drive, or on optical storage devices such as CD-ROM or DVD drives. Alternatively, such files may also be stored on digital media such as a flash memory device.

PPCOMM includes communication software module that is capable of execution and running on a process on the control console. The PPCOMM may be used to establish control of the injector device and maintain data communication with the injector. This module can organize data sequences or messages that are transmitted to the injector on a pre-defined periodic basis. The PPCOMM module can also receive and interpret complimentary data sequences or messages from the injector on a pre-defined periodic basis. PPCOMM may also possesses logic to identify when and if a data transmission problems have occurred. Based upon logic programmed into this module, it may have the ability to intervene and attempt to correct the problem should bi-directional communication remain in-tact. Alternatively, its programmed logic can notify the PPREMOTE application that a communication fault condition has occurred thereby necessitating automatic suspension of injector operation until the problem can be resolved.

PPRESET may include software module that is capable of execution and running on a process on the control console. The PPRESET may provide fault handling and reset capability for the control console.

GINA.DLL may include a dynamic link library that provides system functionality to the control console software elements or modules that are running under an operating system such as Windows, Unix, Linux, MACOS, and the like, and any combination thereof, for example.

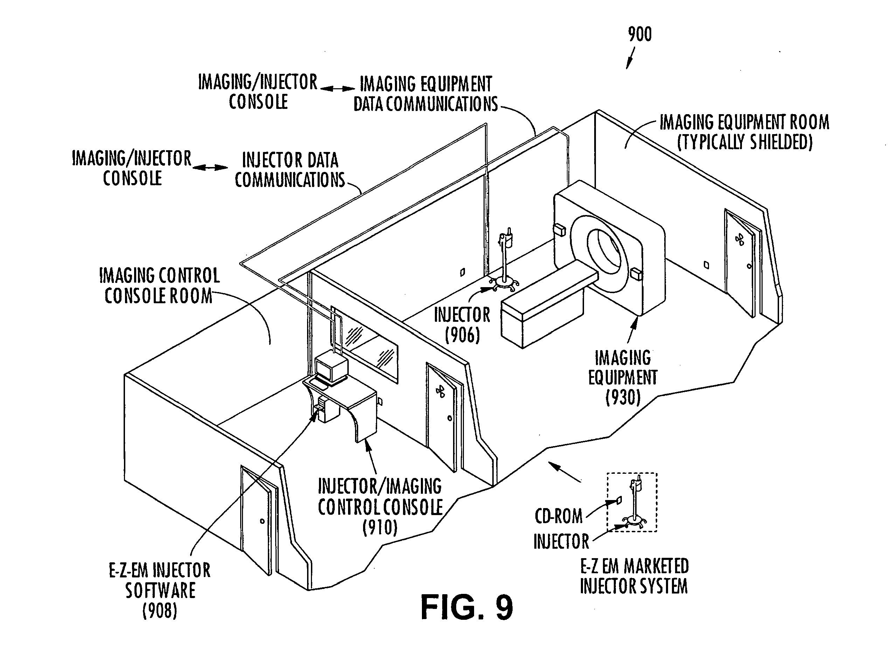

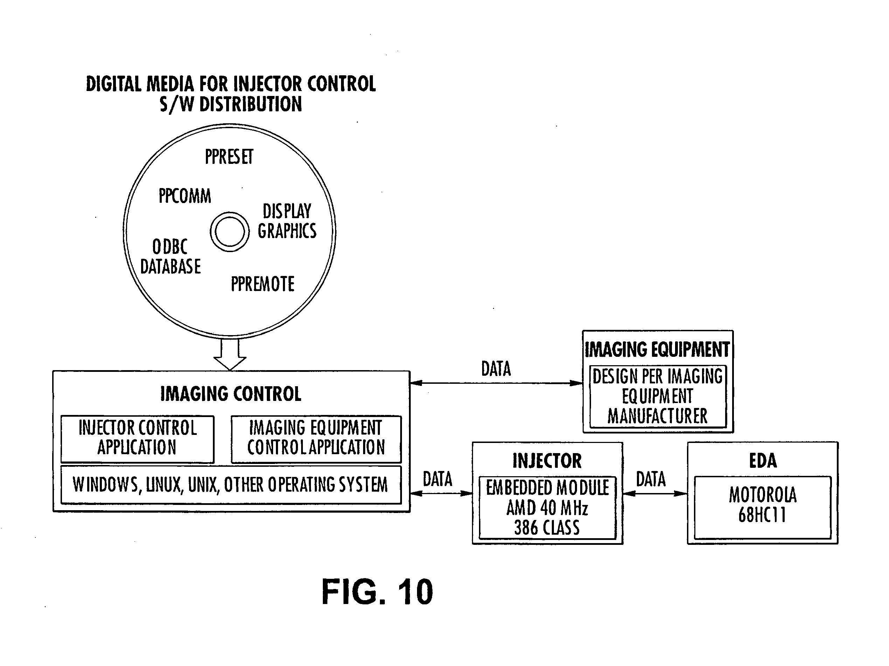

In one alternative example, the above described modules can be packaged and prepared as a software bundle that can be disposed on a transportable digital media (e.g., a CD-ROM, flashcard, etc.). The software may incorporate modules that are necessary for remotely controlling an injector. In one alternative example, it is envisioned that the software can be sold with the injectors so that existing imaging control consoles can be upgraded so that they can be operatively connected with both an injector device and the imaging equipment. In this regard, **FIG. 9** illustrates an imaging suite **900** that has been configured with an injector **906** and the software **908** for remotely controlling the injector with the control console **910.** As a result, both the imaging equipment **930** and the injector device **906** can be monitored and controlled from the injector/imaging control console **910.** **FIG. 10** further illustrates that remote control software can be provided on a storage media, and can be installed in a single computing device for controlling both an injector device and the imaging equipment.

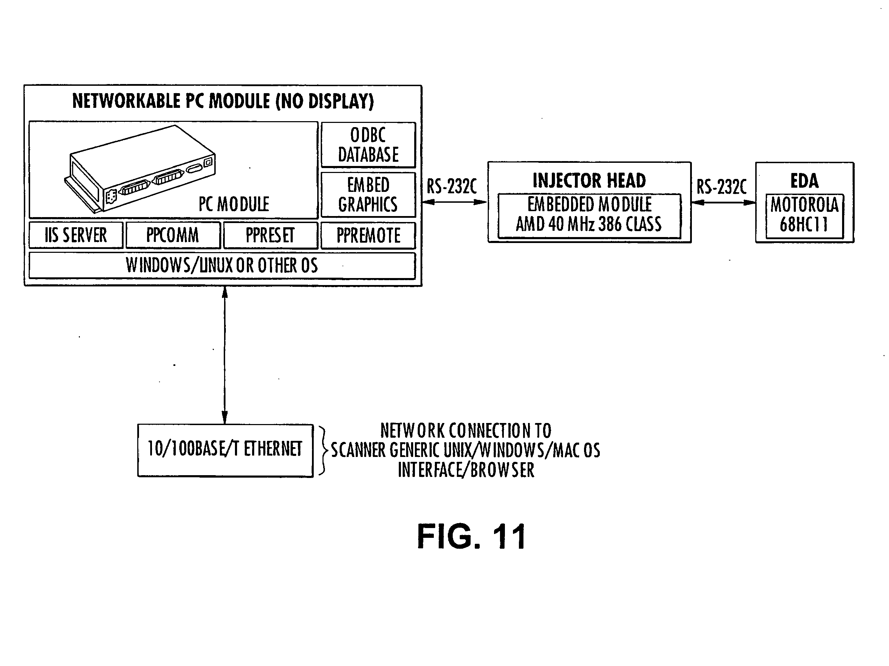

Alternatively, the injector remote software can be used in conjunction with a network capable computer or processing unit, also referred to as a network or PC module. For instance, in one alternative embodiment of the present invention, a processing unit can be included in the injector device, or it can be contained in a standalone enclosure. In other present embodiments, the processing unit may be in communication and controlled by an imaging control console. The imaging control console may communicate with the processing unit via a network connection and protocol. In this regard, **FIG. 11** illustrates an injection device is networked through an exemplary processing unit (networkable PC module), which is in communication with the common control console. In this non-alternative embodiment, the injector device can be in communication with the processing unit/networkable PC module, which is in turn in communication with the common control console. As shown in **FIG. 11****,** the processing unit can be in communication with the common control console through a network connection. In another non-alternative embodiment, the common control console interface can be used to control the injector using network applications such as a browser, or other application. In this embodiment, the processing unit can include the remote software that can contain modules or elements that are necessary to control the injector and send data to and from the common control console. The modules or elements are running on an operating system such as Windows, Linux, Mac OS, Unix, or the like, or any combination thereof.

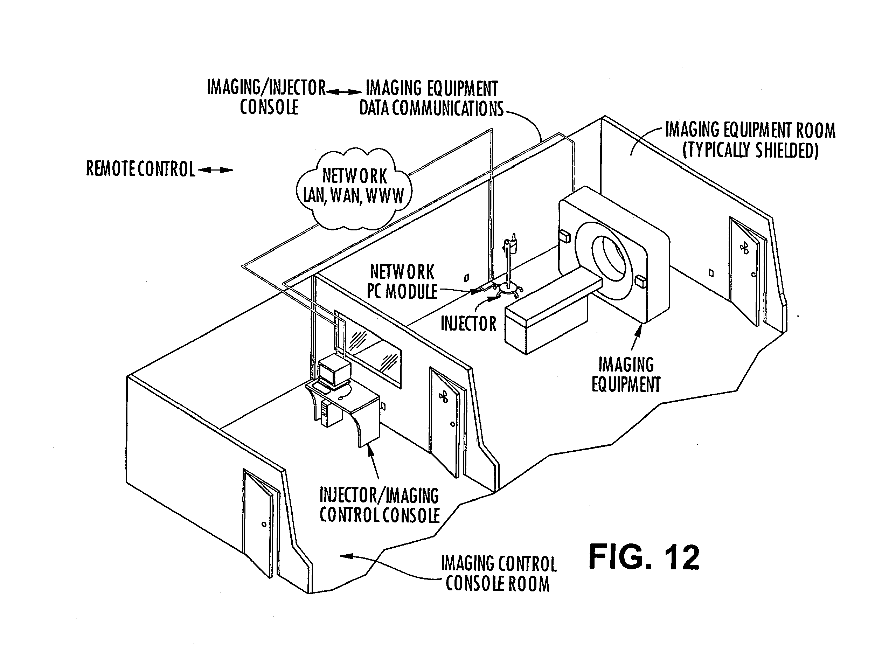

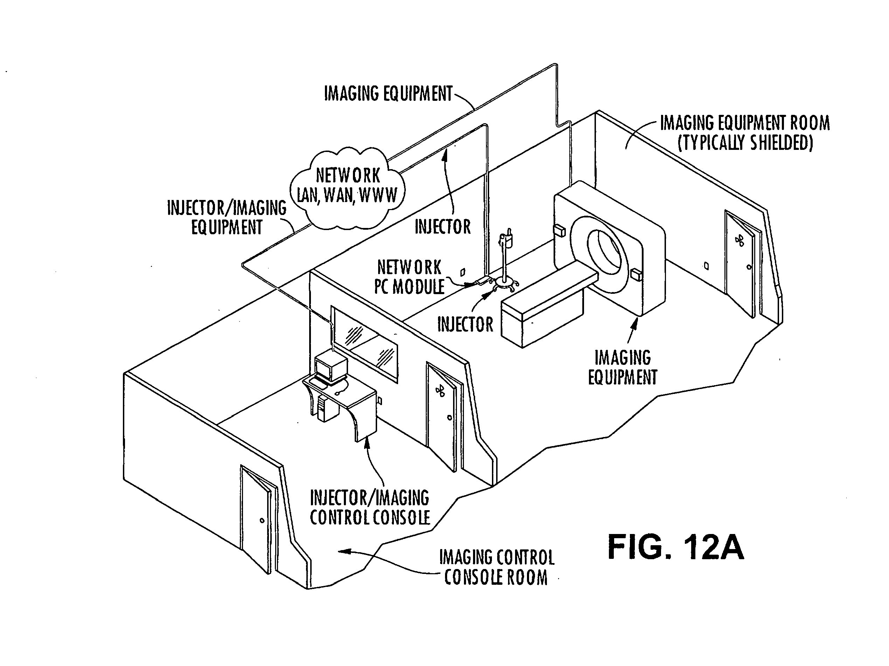

Alternatively, the injector control can be configured as a network client or server. In one alternative embodiment of the present invention, if configured as a client, relevant operational data controlling injector operation can be served from either the imaging control unit directly, or via another server device, proxy, or otherwise in accordance with the invention. If configured as a server, the networkable PC module (see FIG. 12) could serve relevant data from the injector to the imaging control console. As a result, the imaging control console could serve as a common control console for the injector device and imaging equipment.

Additionally, in one example and not part of the present invention, the injector processing unit can also be connected and in communication to the testing facilities internal network, such as, for example, the local hospital network. In this embodiment, the processing unit/networkable PC module may be connected to a local network, and the injection system can be configured as a network appliance within the network. In this configuration, the injector could communicate indirectly with the imaging control station through available network space in the imaging suite. The processing unit connection to the network can be wired or wireless. **Figure 12** further illustrates that an injection system can be controlled by a local network as a network appliance. In this alternative embodiment, the imaging control console using a network connection could serve as a common control console for the injector device and imaging equipment.

In another example, illustrated in **FIG. 12A****,** the injector device and the imaging equipment can share operational parameters via network space as network appliances. In this arrangement, for example, the common control console may derive operational parameters and control information from both the injector and the imaging equipment concurrently. The imaging equipment, injection device, and control console share a common network.

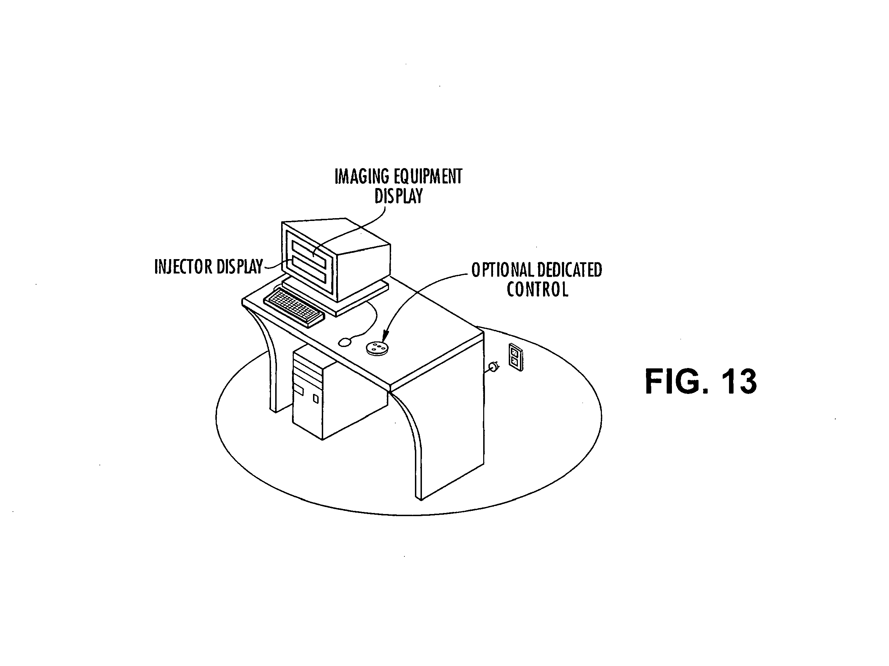

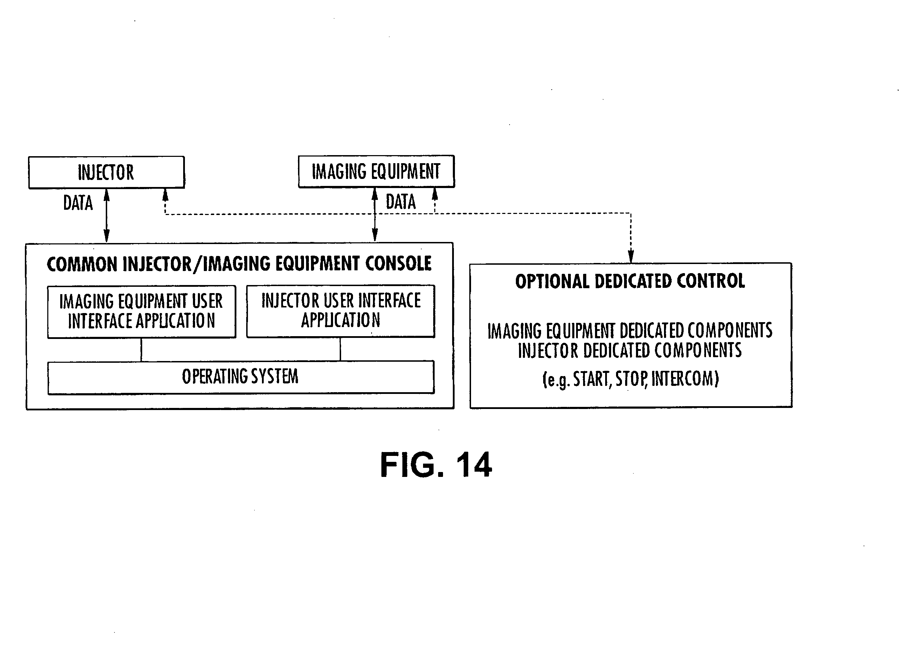

In another alternative embodiment of the present invention, the imaging equipment interface and the injector interface may comprise separate processes that are running within a computer system using a multitasking operating system. In this regard, **FIGS. 13** and **14** illustrate a common control console that is concurrently displaying a dedicated display region for the injector, and a second dedicated display region for the imaging equipment. In this embodiment, the display could be used to simultaneously display applications that are separately in communication with either the injector device or the imaging equipment.

It should be recognized that a variety of different computer platforms and systems could be used in the present invention. The computer platform may include, but is not limited to, a PC or other workstation that is running a graphical user interface (GUI) based operating system such as Windows or Linux, for example. A user interface design may allow the user to freely switch between an injector control application and an imaging control application. The totality of the user interfaces for both the injector device and the imaging equipment can be displayed and managed via a single display, keyboard, pointing device or other commonly available user interface hardware device. The control console and graphical interface can also include a dedicated control console that can be used to have the injector device and imaging equipment perform specific commands. Such commands are known for imaging equipment and include dedicated buttons or keys for frequently used, or safety related operating functions. Such functions include but are not limited to starting, pausing, and stopping the imagery equipment, image recovery, imagery equipment intercom, and the like, and any combination thereof. **FIGS. 13** and **14** illustrate a common control console that also includes one or more dedicated control devices. As shown in **FIGS. 13** and **14****,** a dedicated control device may comprise an interface device that can be used to interface with imagery equipment and the GUI. The system can also include a dedicated control console for frequently used or safety related operating functions of the injection system. Similarly, frequently used or safety related operating functions can be incorporated into a single dedicated control for both the injector device and the imaging equipment. As shown in **FIG. 14****,** the dedicated control console can contain dedicated controls for the injector device, the imaging equipment, or dedicated controls for both the injector device and the imaging equipment.

The dedicated control console can be in communication with the injector device and the imaging equipment in wide variety of manners including, without limitation, a dedicated communication channel that is directly connected to the imaging equipment and injector device, indirect connection via logical interconnection to a common imaging equipment/injector console, and combinations and permutations thereof.

The interface design illustrated in **FIGS. 13** and **14** can be independent processes performed on a common control consoles. The common control console may include a computing platform CPU, memory, I/O, keyboard, display, pointing device, and the like, and any combination thereof. The common control console is associated with input/output device(s) through which the injector device and imaging equipment may be operatively connected. The injector device and imaging equipment may share a common display interface, and they can also be functionally independent from one another. In one embodiment of the present invention, the imaging equipment application may access injector data files running on the common control console. For instance, the common control console can include a software application, such as, for example, EMPOWERCT, which is available from E-Z-EM, that allows the imaging equipment user interface to access injector data, statistics, and other relevant data from databases or similar files, such as the ODBC database file, that are associated with the injector device.

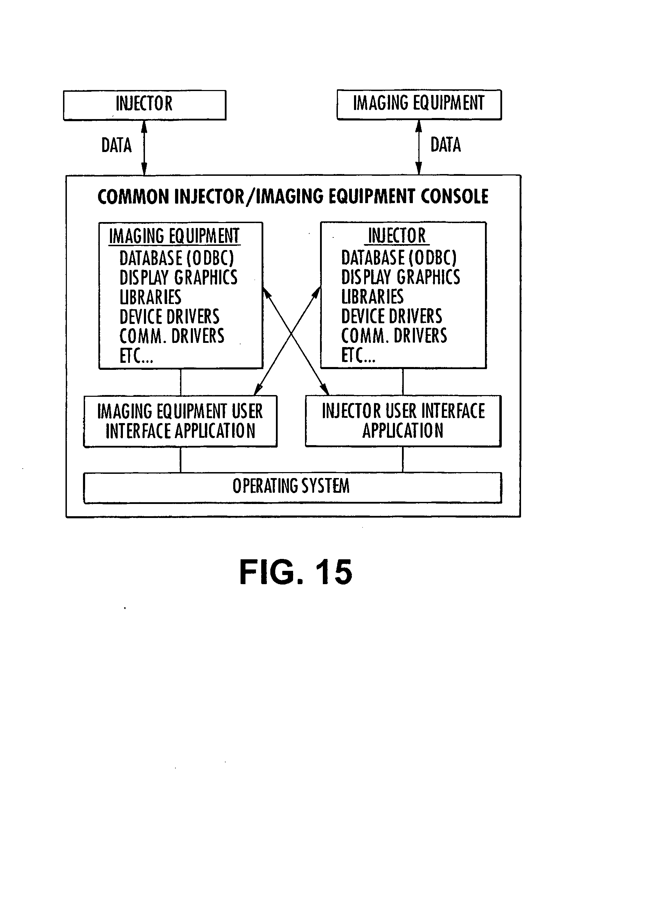

Similarly, in another alternative embodiment of the present invention, if the imaging equipment user interface may comprise a software application that allows it to create, access, and archive imaging equipment data and statistics to a comparable database file, the injector interface application can also access these files. This is one alternative method by which the independent injector and imaging equipment applications could share data amongst themselves for enhancing their respective displays, or supplanting one of them. In this regard, **FIG. 15** illustrates a common control console in which the injector interface application can access files that are associated with the imaging equipment application. In this alternative non-limiting example, the imaging equipment application can also access files associated with the injector device interface.

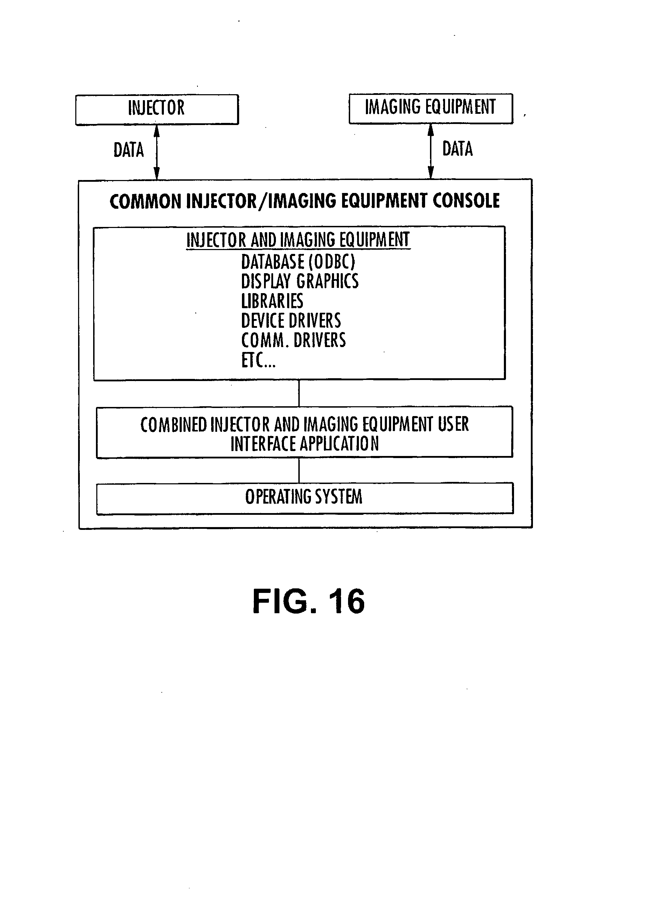

Alternatively, the common control console can include a combined interface application, program, or process that includes both injector and imaging equipment attributes and can be used to control and manage both devices. In this regard, **FIG. 16** illustrates a common control console having a combined interface application that is capable of controlling both the injector device and the imaging equipment. As shown in **FIG. 16****,** the common application program will typically include modules and program elements that are associated with the injector interface and the imaging equipment interface. Such modules may include, for example, database files, display graphics, libraries, device drivers, device specific communication drivers, etc., or any combination thereof.

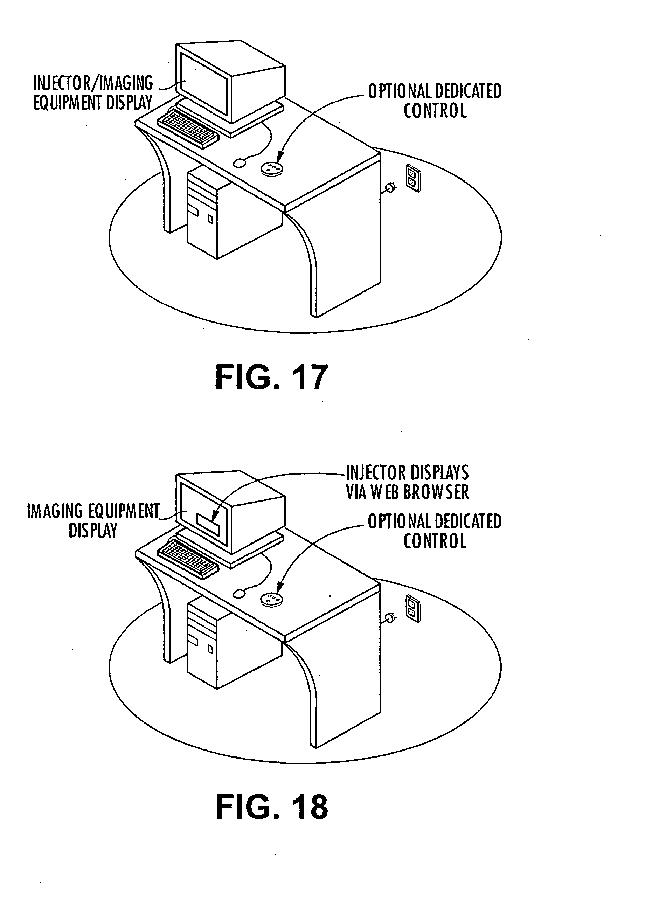

In another alternative embodiment of the present invention, the user interface comprises a single cohesive strategically laid out common user interface that may embody both injector and imaging equipment functions. Thus, remotely controlled injector and imaging equipment functions that require synchronization or any other operational interdependencies can be routinely automated on the common control console. In this regard, **FIG. 17** illustrates a display area of a common injector/imaging equipment console in which a single display window containing both user interface functions of the injector and associated imaging equipment in accordance with one non-limiting embodiment of the present invention.

Alternatively, the injector device and/or imaging equipment interfaces can be configured as a web or network portal. In this alternative, non-limiting embodiment, a generic web browser or dedicated network based application can be used on the common control console to display the injector device and imaging equipment interface. The web browser can be used in a wide variety of ways. For instance, a web browser can be used in conjunction with the network module arrangement that is illustrated in **FIG. 11****.** Alternatively, both the imaging equipment, CPU, and injector, are network appliance devices that can be interconnected via a network protocol. These connections could be peer to peer, LAN, WAN, and/or Internet, for example. Additionally, the connections can be wired or wireless. After a connection is established, the injector user interface display may be displayed on the imaging equipment console on a web browser supporting such standards as HTML, XML, JAVA, .NET, etc., or any combination thereof.

**FIG. 18** illustrates an injection system utilizing a web browser. As shown, the injector user interface is concurrently displayed on a web browser with the imaging equipment application. The imaging equipment interface can also be concurrently displayed on a web browser with an injector application on the common user interface. Alternatively, both the injector and imaging equipment interfaces can be served to two web browser windows on a common processing device with display and input devices. Such a hybrid interface design could accommodate pre-programmed data transfer between an injector interface being served on a web browser and the imaging equipment interface process running directly on the common display interface CPU, or similarly vice-versa.

In one advantageous form of the invention, the system can be comprised of an injector, such as a CT injector, imaging equipment, and a common control console. In this embodiment, the injector operating parameters can be stored and displayed at the user interface. The operating parameters may be manipulated to optimize the imaging and detection data. The specific parameters may be dependent upon the specific media being injected, the part of the subject being imaged, and the like, and any combination thereof. The media typically includes contrast media, saline media, and the like, and any combination thereof. Such operational parameters include, but are not limited to, phases, flow rates, volumes, pressures, timed pauses, hold, and delays to x-ray exposure. The operational parameters for specific tests can be grouped together and stored for later recall. Such parameters can be placed in individual groups as well. These groupings of operational parameters are most commonly called a protocol. In one embodiment of the present invention, stored protocols allow operators to quickly recall optimized parameters that can be used in subsequent tests. As a result, the efficiency of the test and imaging quality can be improved.

Similarly, the operating parameters for the imaging equipment can also be grouped into a protocol for use in subsequent tests. In the case of a CT scanner, such parameters typically include, but are not limited to, kV (voltage applied to an x-ray tube, mA (x-ray tube current) detector collimation, pitch (table speed) gantry rotation speed, detector configuration (number of detector slices number and resultant size), automatic control parameters (dose), timed pauses, holds, and/or delays, and the like, and any combination thereof. The imaging parameters may be displayed on the user interface.

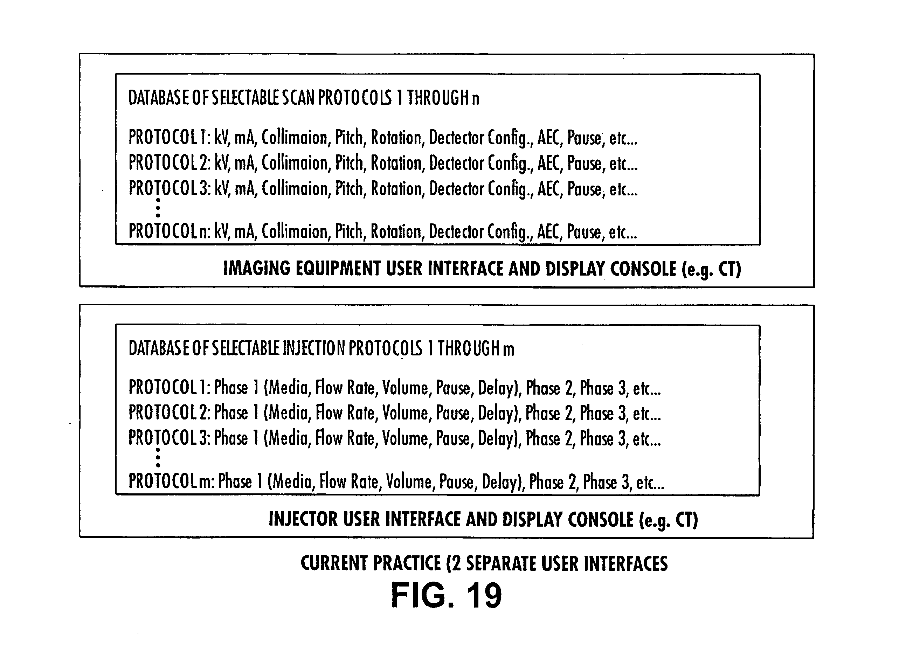

With reference to **FIG. 19** user interface is illustrated that can concurrently display operational parameters for both the injector device and the imaging equipment. As shown in **FIG. 19****,** the user interface may be used to access database files containing various protocols for both the injector device and the imaging equipment. The user interface may be used to allow an operator to easily recall protocols for the injector and the imaging equipment. The operating parameters described above and illustrated in **FIG. 19** include parameters for a CT injection and scanning. It should be understood that the invention is not limited to CT scanning and imagery, and that operation parameters and protocols for a wide variety of other tests can also be used in the practice of the present invention.

For example, in current CT or computed tomography imaging practice whereby two display consoles are used, a clinician performing, for example, a cardiac CT angiography procedure would at one point in the set-up process access the imaging console and another point in time access the injector remote control independently of one another. On the imaging console, the clinician would either manually enter or recall pre-stored CT scan parameters. For a cardiac CT angiography procedure, typical procedure variables for a contemporary 16-slice multi-detector row CT scanner are presented below Table 1.

**Table 1: CT Scanner Parameters**

| CT Scanner Parameters | Values Entered/Stored/Recalled at Imaging Console |
|---|---|
| Tube Current | 150 mAs |
| Tube Potential | 120 Kvp |
| Collimation | 16 slices x 0.625 mm slice thickness |
| Pitch | 1.0 |
| Gantry Rotation | 0.5 sec per Rotation |
| Scan Trigger | Manufacturer Specific |

The above listed CT scanner control parameters are common across various CT scanner manufacturer platforms and the industry in general. While each manufacturer may have several ancillary or special purpose parameters as part of their CT scanner design, the above list should not be considered exhaustive and any other ancillary parameter can easily be included into an imaging console interface design for entering, storing or recalling such parameters. For example, the above grouping of CT scanner parameters could be electronically saved and retrieved under a user named protocol identifier. In this case "Cardiac" could be used to name the protocol on the CT console.

Similarly on the injector remote control, the clinician would either manually enter or recall pre-stored CT injection parameters separate and apart from the imaging console. For a cardiac CT angiography procedure, typical procedure variables for a contemporary two phase contrast injection with saline flush is presented below in Table 2.

**Table 2: CT Injector Parameters**

| CT Injector Parameters | Values Entered/Stored/Recalled at Injector Remote Control |
|---|---|
| Phase 1 Contrast Flow Rate | 4 ml/sec |
| Phase 1 Contrast Volume | 100 ml |
| Phase 2 Saline Flow Rate | 4 ml/sec |
| Phase 2 Saline Volume | 30 ml |
| Pressure | 300 psi |
| Scan Delay | 15 seconds |

The above listed CT injector control parameters are common across various CT injector manufacturer platforms and the industry in general. While each manufacturer may have several ancillary or special purpose parameters as part of their CT injector design, the above list should not be considered exhaustive and any other ancillary parameter can easily be included into an injector remote interface design for entering, storing or recalling such parameters. For example, the above grouping of CT scanner parameters could be electronically saved and retrieved under a user named protocol identifier. In this case, it could use the same name, "Cardiac" that was used to name the protocol o the CT console.

For the proposed practice of acquiring cardiac CT images with a common console serving requirements of both the CT scanner and CT injector, it would be desirable to recall procedure variables for both the CT scanner and CT injector under one unique idenfier. For example, the design and format of a single combined device protocol under a user specified name is facilitated by this invention. For example the common console serving the CT scanner and CT injector could have a named protocol "Cardiac" possessing the aforementioned parameters as follows:

**Table 3: Combined CT Imaging and Scanning Protocol**

| CT Procedure Parameters for Scanning and Contrast Injection | Values Entered/Stored/Recalled at Console Concurrently Servicing CT scanner and CT injector |
|---|---|
| Tube Current | 150 mAs |
| Tube Potential | 120 Kvp |
| Collimation | 16 slices x 0.625 mm slice thickness |
| Pitch | 1.0 |
| Gantry Rotation | 0.5 sec per Rotation |
| Phase 1 Contrast Flow Rate | 4 ml/sec |
| Phase 1 Contrast Volume | 100 ml |
| Phase 2 Saline Flow Rate | 4 ml/sec |
| Phase 2 Saline Volume | 30 ml |
| Pressure | 300 psi |
| Scan Trigger/Scan Delay | Manufacturer Specific |

Design of procedure parameter storage and recall within the interface of a common console for and imaging and injector device in this capacity provides protocol organization, convenience and productivity benefit to the clinician.

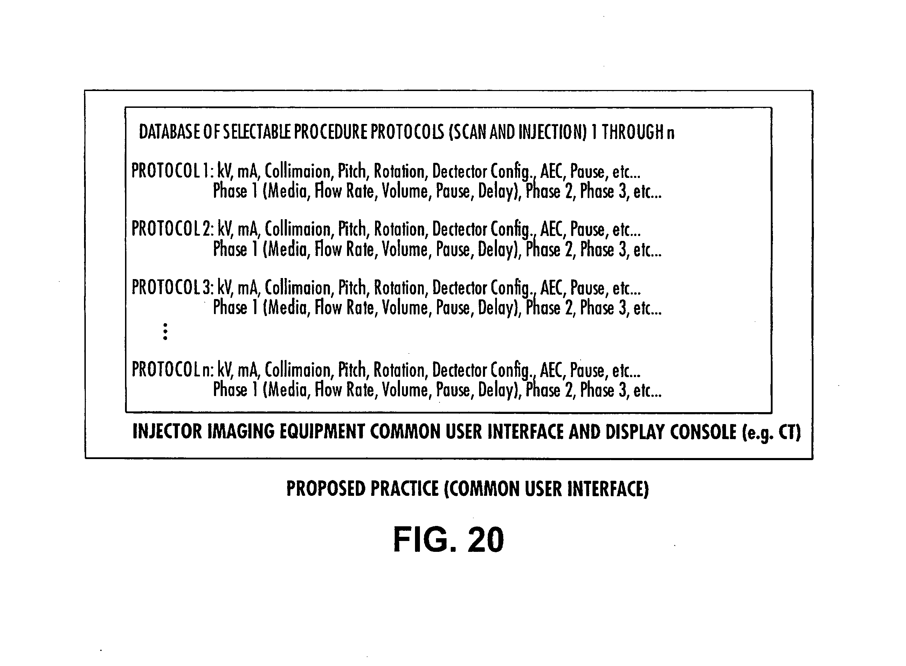

Alternatively, the operational parameters for the injection device and the imaging equipment may be combined into a single protocol. In this regard, **FIG. 20** shows various protocols that contain operational parameters for both the injector device and the imaging equipment. As shown in **FIG. 20****,** the combined protocol can be displayed on a single display. An operator can use a combined protocol to operate the injector device and the imaging equipment. These combined protocols should allow an operator to efficiently recall operation parameters for both injector device and the imaging equipment that have been optimized for a specific test. As a result, the efficiency of the test and the image quality can be improved. In **FIG. 20****,** CT scanning and injection parameters are given for the purpose of example only, and should not be considered as limiting the invention.

The injection/imaging system can be particularly useful for acquiring one or more internal images from within a patient or subject. To acquire the plurality of images, a patient/subject may be placed on a surface, such as a bed, that is in close proximity to an injector device and imaging equipment. The common control console is typically used to select and retrieve from memory desired operational parameters for injecting a contrast medium into the patient. The parameters can be varied by the operator at the interface or alternatively, can be included in a stored protocol that contains a grouping of operational parameters. The operational parameters for the imaging equipment are also typically retrieved or loaded onto the system by the operator. These parameters can also be individually varied and controlled by the operator at the interface, or can be grouped into a stored protocol that can be retrieved from memory or another device. The protocols for both the imaging equipment and the injection device are synchronized so that the injection/imaging system functions cooperatively and concurrently to efficiently perform the testing. Alternatively, a combined protocol containing operational instructions for both the injector device and the imaging equipment can be created and retrieved from memory.

When the patient is ready, the common control console can be used to communicating instructions to the injection device and the imaging equipment. The injection device can inject an effective amount of contrast medium into a patient according to instructions it has received from the common control console. The imaging equipment can scan the patient to acquire internal images. During scanning, the imaging equipment can communicate scanned image data to the common control console where the data can be stored, analyzed, printed, or the like. If desired, the operator can typically control the scanner in a wide variety of ways to obtain the desired images.

Other modifications and other embodiments of the invention set forth herein will come to mind to one skilled in the art to which this invention pertains having the benefit of the teachings presented in the foregoing descriptions and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

Further, throughout the description, where compositions are described as having, including, or comprising specific components, or where processes or methods are described as having, including, or comprising specific steps, it is contemplated that compositions of the present invention also consist essentially of, or consist of the recited components, and that the processes or methods of the present invention also consist essentially of or consist of the recited steps. Further, it should be understood that the order of steps or order for performing certain actions are immaterial so long as the invention remains operable. Moreover, two or more steps or actions may be conducted simultaneously with respect to the invention disclosed herein.

## Claims

1. A system for injecting and imaging a contrast medium in an individual comprising:
a) an injector device (306);
b) an imaging device (330); and
c) a common control console (310) operatively connected to said injector device (306) and said imaging device(330), wherein said common control console (310) is capable of sending and receiving data to and from said injector device (306) and said imaging device (330) so that the operation of said injector device (306) and said imaging device (330) can be controlled from said common control console (310), **characterised in that** said common control console (310) includes a common software application (908) capable of operating the injector device (306) and the imaging device, and said common software application (908) including a plurality of combined device protocols having stored operational parameters for both the injector device (306) and the imaging device (330) grouped together for conducting a specific test.

2. A system according to claim 1, wherein said common control console (310) includes a storage medium for recording data from said injector device (306) and said imaging device (330).

3. A system according to claims 1 or 2, wherein said common control console (310) includes a display unit and an input means.

4. A system according to claim 3, wherein said display unit is a computer monitor, LCD display, plasma display, or television monitor.

5. A system according to claim 3, wherein the common control console (310) includes an injector device control interface and an imaging device control interface capable of displaying concurrently on a single display unit.

6. A system according to claim 5, wherein the injector device control interface is displayed in a first region on said display unit, and the imaging device control unit is displayed in a second region on the display unit.

7. A system according to claims 5 or 6, wherein said common control console (310) includes an injector device application and a separate imaging device application, wherein said injector device application and said imaging device application can be run concurrently.

8. A system according to claim 7, wherein said injector device application is in communication with said imaging device application so that data and/or files can be shared therebetween.

9. A system according to claims 7 or 8, wherein said imaging device application is in communication with said injector device (306) application so that said imaging device application can share data and/or files with said injector device application.

10. A system according to any one of the preceding claims, wherein said common control console (310) comprises a computer system having an operating system that is capable of operating said injector device (306) and said imaging device (330).

11. A system according to any one of the preceding claims, wherein said injector device (306), said imaging device (330), and said common control console (310) are operatively connected through a network.

12. A system according to claim 1, wherein said injector device operational parameters include operational parameters selected from the group consisting of flow rate, media, volume, pressure, phases, KVO, pause, hold, delay, start, and stop.

13. A system according to claim 12, wherein said imaging device operational parameters include operational parameters selected from the group consisting of tube current, tube voltage, collimation, pitch, detector configuration, rotation, pause, scan delay, start, and stop.

14. A system according to claim 1, wherein said plurality of combined device protocols can be created, stored, and recalled on the common control console.

15. A system according to claim 1, wherein the system includes a processing unit operatively connected to said injector device (306) and said common control console (310), wherein said processing unit is capable of sending and receiving data to and from said injector device (306), and said common control console (310) is capable of sending and receiving data to and from said processing unit and said imaging device (330).

16. A system according to any one of the preceding claims, wherein said processing unit is disposed in the injector device (306).

17. A system according to claim 16, wherein said processing unit includes an operating system having remote software that is capable of running on said operating system.

18. A system according to claim 17, wherein the remote software includes the plurality of combined device protocols.

19. A system according to claims 17 or 18, wherein said remote software is capable of controlling said injector device, said software including; PPREMOTE software modules, display graphics software modules, ODBC Database software modules, PPCOMM software modules, PPRESET software modules, and GINA.DLL software modules.

20. A system according to claim 19, wherein said PPREMOTE software module comprises an executable program having program routines for storing, managing and mathematically operating on injector data variables.

21. A system according to claim 20, wherein said PPREMOTE software further comprises program routines to read and write to said ODBC database files.

## Patentansprüche

1. System zum Injizieren und Abbilden eines Kontrastmittels in einer Person, umfassend:
a) ein Injektionsgerät (306);
b) ein Abbildungsgerät (330); und
c) ein gemeinsames Steuerpult (310), das betrieblich mit dem Injektionsgerät (306) und dem Abbildungsgerät (330) verbunden ist, worin das gemeinsame Steuerpult (310) zum Senden und Empfangen von Daten zu und von dem Injektionsgerät (306) und dem Abbildungsgerät (330) imstande ist, sodass der Betrieb des Injektionsgeräts (306) und des Abbildungsgeräts (330) von dem gemeinsamen Steuerpult (310) gesteuert werden kann, **dadurch gekennzeichnet, dass** das gemeinsame Steuerpult (310) eine gemeinsame Softwareanwendung (908) umfasst, die zum Betreiben des Injektionsgeräts (306) und des Abbildungsgeräts imstande ist, und die gemeinsame Softwareanwendung (908) eine Vielzahl von kombinierten Geräteprotokollen einschließt, die Betriebsparameter sowohl für das Injektionsgerät (306) als auch für das Abbildungsgerät (330) gespeichert haben, die zum Vornehmen einer spezifischen Prüfung miteinander gruppiert sind.

2. System nach Anspruch 1, worin das gemeinsame Steuerpult (310) ein Speichermedium zum Aufzeichnen von Daten von dem Injektionsgerät (306) und dem Abbildungsgerät (330) einschließt.

3. System nach Anspruch 1 oder 2, worin das gemeinsame Steuerpult (310) eine Anzeigeeinheit und ein Eingabemittel einschließt.

4. System nach Anspruch 3, worin die Anzeigeeinheit ein Computerbildschirm, eine LCD-Anzeige, eine Plasmaanzeige oder ein Fernsehbildschirm ist.

5. System nach Anspruch 3, worin das gemeinsame Steuerpult (310) eine Injektionsgerät-Steuerschnittstelle und eine Abbildungsgerät-Steuerschnittstelle einschließt, die zum gleichzeitigen Anzeigen auf einer einzigen Anzeigeeinheit imstande sind.

6. System nach Anspruch 5, worin die Injektionsgerät-Steuerschnittstelle in einem ersten Bereich auf der Anzeigeeinheit angezeigt wird und die Abbildungsgerät-Steuereinheit in einem zweiten Bereich auf der Anzeigeeinheit angezeigt wird.

7. System nach Anspruch 5 oder 6, worin das gemeinsame Steuerpult (310) eine Injektionsgerät-Anwendung und eine separate Abbildungsgerät-Anwendung einschließt, worin die Injektionsgerät-Anwendung und die Abbildungsgerät-Anwendung gleichzeitig ausgeführt werden können.

8. System nach Anspruch 7, worin die Injektionsgerät-Anwendung mit der Abbildungsgerät-Anwendung in Kommunikation steht, sodass Daten und/oder Dateien zwischen ihnen gemeinsam genutzt werden können.

9. System nach Anspruch 7 oder 8, worin die Abbildungsgerät-Anwendung mit der Injektionsgerät-(306-)Anwendung in Kommunikation steht, sodass die Abbildungsgerät-Anwendung Daten und/oder Dateien mit der Injektionsgerät-Anwendung gemeinsam nutzen kann.

10. System nach einem der vorhergehenden Ansprüche, worin das gemeinsame Steuerpult (310) ein Computersystem mit einem Betriebssystem, das zum Betreiben des Abbildungsgeräts (330) und des Injektionsgeräts (306) imstande ist, umfasst.

11. System nach einem der vorhergehenden Ansprüche, worin das Injektionsgerät (306), das Abbildungsgerät (330) und das gemeinsame Steuerpult (310) über ein Netzwerk betrieblich verbunden sind.

12. System nach Anspruch 1, worin die Betriebsparameter für das Injektionsgerät Betriebsparameter einschließen, die aus der Gruppe ausgewählt werden, die aus Folgendem besteht: Durchflussrate, Medien, Volumen, Druck, Phasen, KVO, Pause, Halten, Verzögern, Start und Stopp.

13. System nach Anspruch 12, worin die Betriebsparameter für das Abbildungsgerät Betriebsparameter einschließen, die aus der Gruppe ausgewählt werden, die aus Folgendem besteht: Röhrenstrom, Röhrenspannung, Kollimation, Schrittweite, Detektorkonfiguration, Drehung, Pause, Abtastverzögerung, Start und Stopp.

14. System nach Anspruch 1, worin die Vielzahl von kombinierten Geräteprotokollen auf dem gemeinsamen Steuerpult erzeugt, gespeichert und abgerufen werden kann.

15. System nach Anspruch 1, worin das System eine Verarbeitungseinheit einschließt, die betrieblich mit dem Injektionsgerät (306) und dem gemeinsamen Steuerpult (310) verbunden ist, worin die Verarbeitungseinheit zum Senden und Empfangen von Daten zu und von dem Injektionsgerät (306) imstande ist und das gemeinsame Steuerpult (310) zum Senden und Empfangen von Daten zu und von der Verarbeitungseinheit und dem Abbildungsgerät (330) imstande ist.

16. System nach einem der vorhergehenden Ansprüche, worin die Verarbeitungseinheit im Injektionsgerät (306) angeordnet ist.

17. System nach Anspruch 16, worin die Verarbeitungseinheit ein Betriebssystem mit abgesetzter Software einschließt, die imstande ist, auf dem Betriebssystem zu laufen.

18. System nach Anspruch 17, worin die abgesetzte Software die Vielzahl von kombinierten Geräteprotokollen einschließt.

19. System nach Anspruch 17 oder 18, worin die abgesetzte Software zum Steuern des Injektionsgeräts imstande ist, wobei die Software einschließt: PPREMOTE-Softwaremodule, Anzeigegrafik-Softwaremodule, ODBC-Datenbanksoftwaremodule, PPCOMM-Softwaremodule, PPRESET-Softwaremodule und GINA.DLL-Softwaremodule.

20. System nach Anspruch 19, worin das PPREMOTE-Softwaremodul ein ausführbares Programm mit Programmroutinen zum Speichern, Verwalten und mathematischen Bearbeiten von Injektordatenvariablen umfasst.

21. System nach Anspruch 20, worin die PPREMOTE-Software ferner Programmroutinen zum Lesen und Schreiben auf den ODBC-Datenbankdateien umfasst.

## Revendications

1. Système pour injecter et réaliser une imagerie d'un produit de contraste dans un individu comprenant :
a) un dispositif d'injection (306) ;
b) un dispositif d'imagerie (330) ; et
c) une console de commande commune (310) connectée de manière fonctionnelle audit dispositif d'injection (306) et audit dispositif d'imagerie (330), dans lequel ladite console de commande commune (310) est capable d'envoyer ou de recevoir des données vers et à partir dudit dispositif d'injection (306) et dudit dispositif d'imagerie (330) de sorte que le fonctionnement dudit dispositif d'injection (306) et dudit dispositif d'imagerie (330) peut être commandé à partir de ladite console de commande commune (310), **caractérisé en ce que** ladite console de commande commune (310) comprend une application logicielle commune (908) capable de faire fonctionner le dispositif d'injection (306) et le dispositif d'imagerie, et ladite application logicielle commune (908) comprenant une pluralité de protocoles de dispositif combinés ayant des paramètres fonctionnels stockés pour les dispositifs d'injection (306) et d'imagerie (330) qui sont groupés ensemble pour réaliser un essai spécifique.

2. Système selon la revendication 1, dans lequel ladite console de commande commune (310) comprend un support de stockage pour enregistrer des données provenant dudit dispositif d'injection (306) et dudit dispositif d'imagerie (330).

3. Système selon la revendication 1 ou 2, dans lequel ladite console de commande commune (310) comprend une unité d'affichage et un moyen d'entrée.

4. Système selon la revendication 3, dans lequel ladite unité d'affichage est un moniteur d'ordinateur, un affichage LCD, un affichage au plasma, ou un écran de télévision.

5. Système selon la revendication 3, dans lequel la console de commande commune (310) comprend une interface de commande de dispositif d'injection et une interface de commande de dispositif d'imagerie capables d'afficher simultanément sur une unité d'affichage unique.

6. Système selon la revendication 5, dans lequel l'interface de commande de dispositif d'injection est affichée dans une première région sur ladite unité d'affichage, et l'unité de commande de dispositif d'imagerie est affichée dans une seconde région sur l'unité d'affichage.

7. Système selon la revendication 5 ou 6, dans lequel ladite console de commande commune (310) comprend une application de dispositif d'injection et une application séparée de dispositif d'imagerie, dans lequel ladite application de dispositif d'injection et ladite application de dispositif d'imagerie peuvent être mises en oeuvre simultanément.

8. Système selon la revendication 7, dans lequel ladite application de dispositif d'injection est en communication avec ladite application de dispositif d'imagerie de sorte que des données et/ou des fichiers peuvent être partagés entre celles-ci.

9. Système selon la revendication 7 ou 8, dans lequel ladite application de dispositif d'imagerie est en communication avec ladite application de dispositif d'injection (306) de sorte que ladite application de dispositif d'imagerie peut partager des données et/ou des fichiers avec ladite application de dispositif d'injection.

10. Système selon l'une quelconque des revendications précédentes, dans lequel ladite console de commande commune (310) comprend un système informatisé présentant un système d'exploitation qui est capable de faire fonctionner ledit dispositif d'injection (306) et ledit dispositif d'imagerie (330).

11. Système selon l'une quelconque des revendications précédentes, dans lequel ledit dispositif d'injection (306), ledit dispositif d'imagerie (330), et ladite console de commande commune (310) sont connectés de manière fonctionnelle par l'intermédiaire d'un réseau.

12. Système selon la revendication 1, dans lequel les paramètres fonctionnels dudit dispositif d'injection comprennent des paramètres fonctionnels sélectionnés parmi le groupe constitué de débit, média, volume, pression, phases, observation de valeur clé, pause, en attente, retard, démarrage, et arrêt.

13. Système selon la revendication 12, dans lequel les paramètres fonctionnels dudit dispositif d'imagerie comprennent des paramètres fonctionnels sélectionnés parmi le groupe constitué d'intensité de tube, tension de tube, collimation, pas, configuration de détection, rotation, pause, retard de balayage, démarrage, et arrêt.

14. Système selon la revendication 1, dans lequel ladite pluralité de protocoles de dispositif combinés peuvent être créés, stockés, et rappelés sur la console de commande commune.

15. Système selon la revendication 1, dans lequel le système comprend une unité de traitement connectée de manière fonctionnelle audit dispositif d'injection (306) et à ladite console de commande commune (310), ladite unité de traitement étant capable d'envoyer et de recevoir des données vers et en provenance dudit dispositif d'injection (306) et ladite console de commande commune (310) étant capable d'envoyer et de recevoir des données vers et en provenance de ladite unité de traitement et dudit dispositif d'imagerie (330).

16. Système selon l'une quelconque des revendications précédentes, dans lequel ladite unité de traitement est fournie dans le dispositif d'injection (306).

17. Système selon la revendication 16, dans lequel ladite unité de traitement comprend un système d'exploitation présentant un logiciel distant qui est capable de fonctionner avec ledit système d'exploitation.

18. Système selon la revendication 17, dans lequel le logiciel distant comprend la pluralité de protocoles de dispositif combinés.

19. Système selon la revendication 17 ou 18, dans lequel ledit logiciel distant est capable de commander ledit dispositif d'injection, ledit logiciel comprenant : des modules logiciels PPREMOTE, des modules logiciels d'affichage de graphiques, des modules logiciels de base de données ODBC, des modules logiciels PPCOMM, des modules logiciels PPRESET, et des modules logiciels GINA.DLL.

20. Système selon la revendication 19, dans lequel ledit module logiciel PPREMOTE comprend un programme exécutable présentant des routines de programmation pour stocker, gérer et réaliser des opérations mathématiques sur des variables de données d'injection.

21. Système selon la revendication 20, dans lequel ledit logiciel PPREMOTE comprend en outre des routines de programmation pour lire et écrire vers lesdits fichiers de base de données ODBC.
